# EUROPÄISCHE PATENTSCHRIFT

(11) **EP 2 914 188 B1**
(45) Veröffentlichungstag und Bekanntmachung des Hinweises auf die Patenterteilung: **17.08.2016**
(21) Anmeldenummer: 13783842.1
(22) Anmeldetag: 21.10.2013
(51) Int. Cl.: A61B 17/22, A61B 17/3207, A61M 25/00

(54) **VORRICHTUNG ZUM ABLÖSEN WANDSTÄNDIGER THROMBEN AUS EINEM KÖRPERGEFÄSS**
DEVICE FOR DETACHING PARIETAL THROMBI FROM A BLOOD VESSEL
DISPOSITIF POUR DÉTACHER DES THROMBUS PARIÉTAUX D'UN VAISSEAU

(30) Priorität: 05.11.2012 DE 102012021729
(43) Veröffentlichungstag der Anmeldung: 09.09.2015
(73) Patentinhaber: Universitätsklinikum Freiburg, 79104 Freiburg (DE)
(72) Erfinder: HEHRLEIN, Christoph, 79104 Feiburg (DE)
(74) Vertreter: Rösler, Uwe
(86) Internationale Anmeldenummer: PCT/EP2013/003166
(87) Internationale Veröffentlichungsnummer: WO 2014/067631

(56) Entgegenhaltungen:
- WO-A1-00/07657
- WO-A1-01/56644
- WO-A2-2005/018728
- US-A- 5 556 413
- US-A1- 2001 041 899
- US-A1- 2011 166 637

## Beschreibung

### Technisches Gebiet

Die Erfindung bezieht sich auf eine Vorrichtung zum Ablösen wandständiger aus einem Körpergefäß mit einem Katheter, längs dessen Katheterlängserstreckung wenigstens ein Katheterabschnitt vorgesehen ist, dessen zuordenbarer Katheteraußendurchmesser veränderbar ist und in dessen zuordenbaren Katheterwand wenigstens eine die Katheterwand vollständig durchsetzende Wandöffnung eingebracht ist.

### Stand der Technik

Zur interventionellen Therapie von durch gefäßwandständigen Thromben verengten und von Körperflüssigkeiten durchströmten Gefäßen ist eine Vielzahl von Katheterbasierten, medizinischen Instrumenten bekannt, die der schonenden Ablation von an Gefäßinnenwänden alterungs- oder krankheitsbedingten Ablagerungen dienen. Die häufig auch als Thrombektomie-Kathetersysteme bezeichneten medizinischen Instrumente, sehen jeweils eine zur Gewebeablation geeignete Katheterspitze vor, durch die ablatiertes Thrombus-Material zumeist unterstützt mittels eines innerhalb des Katheterlumens vorherrschenden Unterdruckes extrakorporal entsorgt wird.

Hierzu ist aus der US 2010/0087844 A1 ein mechanisches Thrombektomie-Kathetersystem mit einem mehrlumigen Katheter zu entnehmen, der mit Hilfe eines Führungs- und Positionierungsdrahtes relativ zu einer Thrombose positionierbar ist, die zunächst über einen Spülkanal mit einer Lyse-Lösung vorbehandelt wird. Über einen längs des Katheters verlaufenden Instrumentenkanal tritt anschließend ein korbförmig ausgebildetes Schabwerkzeug distalseitig aus dem Katheter und fragmentiert das im Wege von axialer Hin- und Herbewegung das umliegende Thrombus-Material. Über einen weiteren, längs des Katheters vorgesehenen Aspirationskanal können die separaten Thrombus-Materialfragmente gesammelt und extrakorporal verbracht werden.

Im Unterschied zu dem axial beweglichen, korbartig ausgeführten Schabwerkzeug, wie vorstehend erläutert, zeigen die US 2006/0074441 A1 sowie US 2011 /0040314 A1 drahtförmig ausgebildete Thrombus-Material-Abtragewerkzeuge, die jeweils an einer drahtförmig ausgebildeten Werkzeugspitze eine sinusoidale Drahtform besitzen, die bei Rotation um die Drahtlängsachse die Wirkung einer spindelförmigen Schneidstruktur annimmt und bei Inkontakttreten mit Gefäßwandadhäsivem Thrombus-Material dieses aufgrund rotatorischer Scherkräfte zu zerkleinern und abzutragen vermag. Losgelöste Thrombus-Materialfragmente werden auch hier mit einem geeignet platzierten Aspirationskatheter extrakorporal verbracht.

Ein weiteres, Thrombus-Material ablatierendes Werkzeug ist in der US 2006/0064073 A1 beschrieben, das einen durch einen Aspirationskatheter geführten Werkzeugkatheter betrifft, an dessen distalen Katheterbereich ein radial zur Katheterachse keilförmig abspreizbares Ablationsmittel angebracht ist, das durch rotatorische Bewegung um die Katheterachse intravaskuläres Thrombus-Material abzutrennen und zu fragmentieren vermag. Durch die keilförmig ausgerichteten Werkzeugschenkel und der vermittels des Aspirationskatheters vorherrschenden Saugwirkung gelangen die fragmentierten Thrombosematerialpartikel proximalwärts zur Werkzeugkatheterspitze in den Aspirationskatheter. Eine vorteilhafte Ergänzung zu dem vorstehend beschriebenen mechanischen Thrombektomie-Kathetersystem ist in der US 2006/0253145 A1 beschrieben, in der am distalen Ende des Werkzeugkatheters ein inflatierbarer Ballon vorgesehen ist, der zum einen den Werkzeugkatheter axial innerhalb des Gefäßes und somit relativ zum abzutragenden Thrombus-Material zentriert und andererseits dafür sorgt, dass keine von der Gefäßwand separierte Fragmente von Thrombus-Material distalseits zum fächerförmig aufgeweiteten und rotierenden Abtragewerkzeug unkontrolliert in die der Blutbahn gelangen können.

Die US 2007/0208361 A1 beschreibt ein Atherektomie-Kathetersystem, das einen Aspirationskatheter vorsieht, der distalseitig von einem radial aufweitbaren Stentgeflecht sowie einer zentrisch geführten Nadel überragt ist, die gemeinsam mitrotierend gelagert sind und vergleichbar eines Bohrkopfes durch distalseitigen Vorschub längs eines stenösen Blutgefäßes Thrombus-Material punktiert, separiert und zerkleinert. Die fragmentierten Thrombus-Materialstücke werden über den Aspirationskatheter extrakorporal verbracht.

Eine weitere Art zur lokalen Abtrennung stenosierter Gewebebereiche stellen Kathetersysteme mit Schneidwerkzeugen dar. Aus der US 2009/0270800 A1 ist ein Katheter zu entnehmen, an dessen distalen peripheren Außenbereich in Umfangsrichtung einzelne Blähkörper angeordnet sind, um die axiale Lage der distalen Katheterspitze relativ zu einem zu behandelnden Gefäß individuell zu positionieren. Ferner ist innerhalb des Katheters eine über die Katheterspitze führbare Schneidklinge vorgesehen, mit deren Hilfe Thrombus-Material von der Gefäßwand abtrennbar ist. Abgetrennte Thrombus-Materialstücke gelangen anschließend über einen Aspirationskanal längs der Katheteranordnung.

Eine vergleichbare Katheteranordnung ist der WO 2010/132748 A1 zu entnehmen, die ebenfalls ein Schneidwerkzeug vorsieht, das sowohl axial durch eine radial aufspreizbare Katheterspitze als auch über eine seitlich längs der Katheteranordnung vorgesehene Öffnung führbar gelagert ist.

Aus der US 2008/0300532 A1 ist ein Fluid-durchströmter Thrombektomie-Katheter zu entnehmen, der mit Hilfe eines Führungsdrahtes längs einer stenösen Gefäßstelle positionierbar ist und längs eines distalen Katheterabschnittes über wenigstens zwei quer zur Katheterlängserstreckung orientierte Öffnungen verfügt, von denen eine Öffnung als Fluidaustrittsöffnung dient, aus der ein Fluidstrom quer zur Gefäßlängserstreckung auf das abzutragende Thrombus-Material auftrifft und dieses lokal von der Gefäßinnenwand unter Ausbildung kleinster Thrombus-Materialfragmente ablöst. Die abgelösten Thrombusfragmente gelangen über die zweite Öffnung in das Innere des Katheters, längs dem die mit Thrombus-Material durchsetzte Fluidströmung extrakorporal nach außen gelangt.

Schließlich beschreibt die DE 10 2009 017 050 A1 eine Vorrichtung zum Ablösen von Konkrementen aus einem Körpergefäß mit einem Katheter, durch den ein Werkzeugkatheter relativ zu einer abzutragenden Thrombose positionierbar ist. Der Werkzeugkatheter weist ein stentförmig ausgebildetes Fängerelement auf, das sich nach distalseitigem Ausbringen aus dem Katheter radial selbständig aufweitet. Das Fängerelement verfügt über als Längsschlitze ausgebildete Eintrittsöffnungen, durch die das zu entfernende Konkrementmaterial, insbesondere Thromben, in das Innere des Fängerelementes eindringen. Durch axiale sowie auch rotatorische Bewegungen des Werkzeugkatheters kommt es aufgrund auftretender Scherkräfte zum Ablösen des durch die Eintrittsöffnungen in das Innere des Fängerelementes ragenden Thrombus-Materials, das in Form fragmentierter Einzelstücke durch Applikation eines innerhalb des Werkzeugkatheters anliegenden Unterdruckes extrakorporal verbracht werden kann. Zur Entnahme der Katheteranordnung aus dem Gefäß wird das Fängerelement proximalwärts durch radial wirkende Kompressionskräfte in den Kanal gezogen und in diesem komprimierten Zustand extrakorporal entnommen.

Alle vorstehend bekannten Lösungen stellen technisch zum Teil aufwendige Katheterausbildungen dar, deren operative Handhabung hohe Ansprüche an den Operateur stellen, zumal die zumeist scharfkantigen Werkzeuge zum lokalen intravasalen Abtrag wandständiger Thromben mit großer Vorsicht zu bedienen sind, um Verletzungen an Gefäßwänden zu vermeiden.

Die in der Druckschrift US 7 063 671 B2 offenbarten Vorrichtungen zur Entnahme von Proben bzw. Entfernung von Polypen aus einem Körper eines Patienten weisen Aktoren aus elektroaktiven Polymermaterial auf, die per elektrische Stimulation kontrahiert bzw. expandiert werden können. Durch entsprechende Betätigung der Aktoren können Öffnungen in der Vorrichtung erweitert werden, um eine Probe oder den Polyp zu umfassen.

In der Druckschrift US 2010/0125239 A1 ist als Alternative zu schneidenden, ablatierenden oder verdampfenden Behandlungsverfahren von Stenosen, ein Katheter mit einem aufblasbaren Ballon erläutert, mit dem Medikamente an eine bestimmte Stelle innerhalb eines Lumens, z.B. einer Arterie, appliziert werden können. So weist der Katheter einen Ballon auf, der von einer porösen Membran umgeben ist. Im Zwischenraum zwischen Ballon und Membran befindet sich ein Medikament. Durch Ansteuern von auf der Membran befindlichen Elektroden und die dabei zugeführte Wärmeenergie öffnen sich die Membranporen und Moleküle eines Medikaments werden durch den mit dem Ballon erzeugten Druck durch die Membranporen zum zu behandelnden Gewebe gedrückt. Der Ballon bewirkt auch eine radiale Expansion, bei der der Katheter mit dem umgebenden Gewebe in Kontakt tritt.

Eine im Vergleich zu den vorstehenden, bekannten Katheterlösungen kostengünstigere und für den Operateur leichter zu bedienende Kathetervorrichtung zum Ablösen gefäßwandständiger Thromben geht aus der deutschen Patentanmeldung DE 10 2011 120 004 B3 und nutzt die elastischen Eigenschaften eines aus einem Elastomer bestehenden, schlauchförmigen Katheters, in dessen Katheterwand die Katheterwand vollständig durchdringende Wandöffnungen eingebracht sind, die mit Hilfe eines längs des Katheters einführbaren Verdrängungskörpers durch radiale Dehnung des schlauchförmigen Katheters in einen aufgeweiteten Öffnungszustand überführbar sind. In den Bereich der aufgeweiteten Katheterwandöffnungen dringt wandständiges Thrombenmaterial bei geeigneter intravaskulärer Positionierung des Katheters ein, das nach Entfernen des Verdrängungskörpers aus dem Katheter und einer damit einhergehenden elastischen Rückformung des aufgeweiteten Katheters in seinen ursprünglichen Formzustand innerhalb der sich verkleinernden Katheterwandöffnungen unter Ausbildung von Scherkräften fest eingeklemmt und nachfolgend durch extrakorporale Entnahme des Katheters entfernt wird.

Aus US 2005/0080430 A1 ist ein Katheter mit einem aufweitbaren, distalen Ende bekannt, mit dem eine vor dem Katheterende in einem Körperlumen befindliche medizinische Vorrichtung, die einen größeren Durchmesser als der Katheter aufweist, entfernt oder an einen anderen Ort innerhalb des Körpers verbracht werden kann. Das Ende des Katheters weist Schlitze auf, die zumindest teilweise nicht parallel zur Längserstreckung des Katheters verlaufen. Der geschlitzte Bereich wird durch eine an der Außenseite angeordnete dehnbare, elastomere Schicht zusammengehalten.

Die Druckschrift US 2001/041899 A1 offenbart eine Vorrichtung entsprechend dem Oberbegiff des Anspruchs 1.

### Darstellung der Erfindung

Der Erfindung liegt die Aufgabe zugrunde, eine Vorrichtung zum Ablösen gefäßwandständiger Thromben aus einem Körpergefäß möglichst einfach, kostengünstig und für den Operateur leicht bedienbar auszugestalten, so dass eine Gefäßwandverletzung weitgehend ausgeschlossen werden kann. Zudem gilt es, Gefäßwand-ständiges Thrombus-Material sicher und effizient, d.h. möglichst rückstandsfrei, von der Gefäßwand zu entfernen und die von der Gefäßwand separierten Thrombus-Fragmente sicher extrakorporal zu verbringen.

Die Lösung der der Erfindung zugrunde liegenden Aufgabe ist im Anspruch 1 angegeben. Merkmale, die die lösungsgemäße Vorrichtung in vorteilhafter Weise weiterbilden, sind Gegenstand der Unteransprüche sowie der weiteren Beschreibung, insbesondere unter Bezugnahme auf die Ausführungsbeispiele zu entnehmen.

Ausgehend von der in der deutschen Patentanmeldung DE 10 2011 120 004 B3, zu der die nun vorliegende Anmeldung eine Zusatzanmeldung darstellt, nutzt die lösungsgemäße Vorrichtung gleichsam die elastischen Eigenschaften und die damit verbundenen elastischen Rückstellkräfte eines aus einem elastisch verformbaren Material bestehenden Katheterabschnittes zu Zwecken der Aufnahme sowie des Abtrennens von wandständigem Thrombenmaterial aus einem Körpergefäß, jedoch entfällt die Notwendigkeit eines im vorstehenden, bekannten Fall erläuterten Verdrängungskörpers, wodurch die neuartige Kathetervörrichtung noch einfacher im Aufbau sowie in der Handhabung und auch kostengünstiger realisierbar ist.

Die neuartige Vorrichtung zum Ablösen wandständiger Thromben aus einem Körpergefäß mit einem Katheter, längs dessen Katheterlängserstreckung wenigstens einen Katheterabschnitt vorgesehen ist, in dessen zuordenbaren Katheterwand wenigstens eine die Katheterwand quer zur Katheterlängserstreckung vollständig durchsetzende Wandöffnung vorgesehen ist, zeichnet sich dadurch aus, dass die Katheterwand längs des wenigstens einen Katheterabschnitts aus einem elastisch verformbaren Material besteht, und die wenigstens eine Wandöffnung in Form eines die Katheterwand wenigstens abschnittsweise längs des Katheterabschnitts helikal umlaufenden Trennspaltes ausgeführt ist. Der vorzugsweise über die gesamte axiale Erstreckung des Katheterabschnittes helikal umlaufende Trennspalt ist vorzugsweise als ein die Katheterwand vollständig durchtrennender Einschnitt ausgebildet, d.h. zur Ausbildung des Einschnittes wurde kein Katheterwandmaterial abgetragen, so dass sich die längs des helikal umlaufenden Trennspaltes verlaufenden, einander zugewandten Katheterwandschnittflächen flächig berühren.

Distalseitig zu der wenigstens einen als helikal umlaufenden Trennspalt ausgebildeten Wandöffnung sieht der Katheter ein Fixiermittel vor, das eine vorübergehende, d.h. wieder lösbare Fixierung des Katheters an oder innerhalb eines Körpergefäßes ermöglicht. Das Fixiermittel ist insbesondere derart ausgebildet, so dass der Katheter möglichst drehfest um seine Katheterlängsachse intrakorporal verankerbar bzw. fixierbar ist.

In einer bevorzugten Ausführungsform ist das Fixiermittel als dillatierbarer Ballon ausgebildet, der fest, vorzugsweise an der distalen Katheterspitze oder zumindest distalseitig zum wenigstens einen helikal umlaufenden Trennspalt längs des Katheters angebracht ist und über ein innerhalb des Katheters geführtes Lumen durch Befüllen mit einem geeigneten gasförmigen oder flüssigen Medium dillatierbar ist. Alternativ zu einer festen Anbringung eines Ballons am Katheter ist es ebenso möglich, einen separat zum Katheter handhabbaren Ballonkatheter vorzusehen und diesen durch das innere Lumen des Katheters distalseitig vorzuschieben, bis der Ballon am distalen Ende des Katheters positioniert ist oder diesen distalseitig überragt. Durch entsprechende Inflatierung vermag der Ballon den Katheter innerhalb eines Gefäßes distalseitig fest zu fixieren, so dass der Katheter insbesondere drehfest relativ zu seiner Katheterlängsachse einseitig endseitig fixiert ist.

Selbstverständlich ist es möglich und denkbar das Fixiermittel anderweitig auszubilden, so bspw. in Form von Verankerungsstrukturen, die am distalen Bereich des Katheters dynamisch aus dem Katheter ausfahrbar oder ausklappbar ausgebildet sind. Derartige Verankerungsstrukturen sind dem Fachmann hinlänglich bekannt und bedürfen daher keiner detaillierten Beschreibung.

Der vorstehend erläuterte lösungsgemäße Katheter wird zu Zwecken einer schonenden intravaskulären Ablation von wandständigem Thrombenmaterial innerhalb eines Körpergefäßes derart positioniert, so dass der den wenigstens einen helikal umlaufenden Trennspalt aufweisende Katheterabschnitt unmittelbar längs einer intravasalen, wandständigen Thrombenablagerung zum liegen kommt. In dieser Lage gilt es den Katheter vorzugsweise distal endseitig mit Hilfe des Fixiermittels derart zu fixieren, so dass der Katheter zum einen axial längs des Gefäßes aber insbesondere drehfest fixiert ist. In diesem Zustand gilt es über einen extrakorporal zugänglichen Katheterabschnitt einen äußeren mechanischen Zwang auf den Katheter in Form eines längs des Katheters torsional einwirkenden Drehmomentes derart auszuüben, so dass die dem Drehmoment zugrunde liegende Drehrichtung entgegen der dem helikal umlaufenden Trennspalt zuordenbaren Windungsrichtung orientiert ist. Das Drehmoment kann durch manuelles Verdrillen des Katheters oder unter Zuhilfenahme eines am proximalen Ende des Katheters geeignet angebrachten elektromotorischen Drehantriebes in dosierter Form erzeugt werden.

Durch die distal endseitige Fixierung des Katheters mit Hilfe des Fixiermittels, das das Drehmoment endseitig aufnimmt, vermag das längs des Katheters wirkende Drehmoment den Katheterabschnitt, längs dem sich der wenigstens eine helikal umlaufende Trennspalt erstreckt, von einem Zustand mit einem relativ kleineren Katheteraußendurchmesser in einen Zustand mit relativ größeren Katheteraußendurchmesser im Wege einer elastischen Formänderung der Katheterwand zu überführen, wobei sich der helikal umlaufende Trennspalt von einem geschlossenen Spaltzustand in einen geöffneten Spaltzustand aufweitet. Durch die radiale Aufweitung des Katheterabschnittes gelangt die Katheteraußenwand in Presskontakt zu wandständigen Thromben, die zumindest teilweise in den Zwischenraum des geöffneten Trennspaltes hineinragen.

Nach Abstellen des äußeren mechanischen Zwanges, d. h. nach Beseitigen des auf den Katheter torsional einwirkenden Drehmomentes vermag der Katheter durch materialinhärente, elastische Rückstellkräfte den Zustand mit dem relativ kleineren Katheteraußendurchmesser selbständig wieder einzunehmen, wodurch der zwangsgeöffnete Trennspalt in den anfänglichen, geschlossenen Spaltzustand überführt wird. Durch die Spaltbreitenreduzierung wird innerhalb des Trennspaltes hineinragendes Thrombusmaterial regelrecht eingeklemmt und letztlich von übrigen wandständigen Restmaterial abgeschert.

Die Thrombusmaterialabscherung kann in vorteilhafter Weise dadurch unterstützt werden, indem längs des Katheters ein in Windungsrichtung des helikal umlaufenden Trennspaltes orientiertes Drehmoment proximalseitig eingeleitet wird, wodurch die den helikal umlaufenden Trennspalt jeweils beidseitig begrenzenden Spaltschnittflächen mit einer zusätzlichen Kraft gegeneinander gepresst und somit die auf das Thrombusmaterial gerichteten Abscherkräfte erhöht werden können.

Zu Zwecken einer möglichst vollständigen Erfassung, Ablation und letztlich extrakorporalen Entnahme von wandständigem Thrombusmaterial aus einem Körpergefäß ist es vorteilhaft längs des Katheters, d. h. längs des von dem Katheter umschlossenen Lumens, so insbesondere längs des mit dem wenigstens einen helikal umlaufenden Trennspalt versehenen Katheterabschnittes, einen Unterdruck zu applizieren, der das wandständige Thrombenmaterial durch den Trennspalt im geöffneten Zustand in das Innere des Katheterabschnittes zu saugen und dort auch im wieder geschlossenen Zustand des Katheters sicher zu verwahren vermag. Der Katheter sieht in einer Ausführungsvariante hierzu an seinem proximalen Katheterende eine Anschlussstruktur zur fluiddichten Verbindung an eine geeignete Unterdruck-, bzw. Aspirationsquelle vor.

Selbstverständlich ist es möglich innerhalb der Katheterwand längs des Katheterabschnittes, die vorzugsweise aus einem elastisch verformbaren Elastomer besteht und rohr- bzw. schlauchförmig ausgebildet ist und somit ein inneres Katheterlumen umfasst, zwei, drei oder mehr helikal umlaufende Trennspalte mit jeweils gleich orientierter Windungsrichtung vorzusehen. Die Helizität des wenigstens einen Trennspaltes ist vorzugsweise mit einer einheitlich konstanten Steigung ausgeführt, die sich vorzugsweise über die gesamte Länge des Katheterabschnittes erstreckt. Gleichwohl ist es möglich längs des Katheterabschnittes wenigstens zwei axiale Bereiche vorzusehen, innerhalb der der helikal umlaufende Trennspalt jeweils über unterschiedliche Steigungen verfügt. Gilt es bspw. möglichst große Mengen an wandständigem Thrombusmaterial von einem Gefäßwandbereiches abzulösen, so eignet sich ein lösungsgemäß ausgebildeter Katheter mit wenigstens einem helikal umlaufenden Trennspalt, dessen Steigung möglichst klein gewählt ist, d.h. möglichst viel Windungen sind innerhalb eines axialen Bereiches längs des Katheterabschnittes vorgesehen.

Eine weitere vorteilhafte Ausführungsform sieht längs des Katheterabschnittes zusätzlich zu dem wenigstens einen helikal umlaufenden Trennspalt wenigstens eine die Katheterwand vollständig durchsetzende Wandöffnung vor, die im Unterschied zur Ausbildung des wenigstens einen helikal umlaufenden Trennspaltes durch eine Materialausnehmung aus der Katheterwand gebildet ist und vorzugsweise eine größte Öffnungsweite von 0,1 bis 20 mm aufweist. Die wenigstens eine weitere Wandöffnung behält ihre Öffnungsgeometrie, die vorzugsweise rund, oval, n-eckig ausgebildet ist oder eine hiervon abweichende Öffnungsform aufweist, weitgehend unverändert bei, selbst im Falle einer vorstehend erläuterten Formänderung des Katheters bedingt durch ein auf diesen einwirkendes Drehmoment.

Die wenigstens eine weitere Wandöffnung dient als Aspirationsöffnung im Falle eines innerhalb des Katheters umschlossenen Lumens applizierten Unterdruckes, durch die wandständiges Thrombusmaterial eingesaugt werden kann, vor allem in vorstehend beschriebenen geschlossenem Katheterzustand, den der Katheter während einer intrakorporalen Einführung und Positionierung sowie auch einer nachfolgenden extrakorporalen Entnahme einnimmt.

Sowohl der Ablationsvorgang von wandständigem Thrombusmaterial vermittels des wenigstens einen helikal umlaufenden Trennspaltes sowie auch vermittels der zusätzlich innerhalb der Katheterwand längs des Katheterabschnittes vorzusehenden Wandöffnungen kann in einer bevorzugten weiteren Ausführungsform des lösungsgemäßen Katheters durch axiale und/oder radiale Relativbewegungen des Katheters relativ zur stenosierten Gefäßstelle unterstützt werden. Die Relativbewegungen können entweder manuell durch den Operateur vorgenommen oder mit Hilfe einer geeignet proximalseits am Katheter vorgesehenen Vibratoreinheit unterstützt werden.

Grundsätzlich sieht das lösungsgemäße Kathetersystem keinerlei die gesunde Gefäßwand möglicherweise verletzende Schneidwerkzeuge vor, die getrennt zum Katheter intravasal zu führen sind, wie dies vom einschlägigen Stand der Technik der Fall ist. Vielmehr ermöglicht das lösungsgemäße Kathetersystem eine schonende und vollkommen verletzungsfreie Thrombus-Materialablation von der Gefäßinnenwand, zumal bei Erreichen der gesunden Gefäßinnenwand durch den Katheter das gesunde Gefäßwandmaterial parallel zur Katheteraußenwand anliegt und nicht in den aufgeweiteten Trennspalt hineinzureichen vermag. Der mit wenigstens einer seitlichen Trennspaltöffnung sowie gegebenenfalls mit zusätzlichen Wandöffnungen versetzte Katheter vermag somit lediglich Gefäßwand-adhärentes Gewebematerial abzutragen, das über die Gefäßwandoberfläche hinausragt.

Um wandständiges Thrombus-Material vollständig zu beseitigen, bedarf es zumeist einer mehrmaligen hintereinander durchzuführenden Positionierung des Katheters relativ zum intravasal vorhandenen Thrombus-Material und den vorstehend beschriebenen Abtrennvorgang. Zwar ist es möglich, den Katheter nach jeder einzelnen Gewebeabtrennung durch einen ebenfalls intrakorporal eingeführten Arbeitskatheter proximalwärts zurückzuziehen und entsprechend extrakorporal von dem abgetrennten Gewebematerial zu säubern, um den Katheter erneut intrakorporal zu positionieren, doch ist es in Fällen, in denen größere Mengen an Thrombus-Material an einer Stenose abzutragen ist, vorteilhaft, proximalseits am Katheter eine Unterdruckquelle anzuschließen, über die abgetrenntes Thrombusmaterial innerhalb des Katheters proximalseitig extrakorporal verbracht werden kann.

Die Katheterwand des Katheters, insbesondere im Bereich des Katheterabschnittes besteht aus einem biokompatiblen Elastomer, das aus wenigstens einem Material aus den nachfolgend aufgeführten Materialien besteht:
Polymethylmethacrylat (PMMA), Polytetrafluoroethylen (PTFE), Polyurethane, Polyetherurethane, Silicon-Polyetherurethane, Silicon-Polyurethane, Silicon-Polycarbonat-Urethane, Polyolefin-Elastomere, Polylsobutylene,
Fluorosilicone,Polyvinylchlorid (PVC), Polydimethylsiloxan (PDMS), Polylactide, Polyethylen,Polybutylmethacrylat, Polyacrylamid, Polyacrylonitrile, Polyamide, Polyetheramide,Polyethylenamin Polyimide, Polycarbonate, Polycarbourethane, Polyvinylketone, Polyvinylhalogenide, Polyvinylidenhalogenide, Polyvinylether, Polyisobutylene, Polyvinylaromaten, Polyvinylester, Polyvinylpyrollidone, Polyoxymethylene, Polytetramethylenoxid, Nylon oder Polyester

Die vorstehenden Materialien ermöglichen eine einfache und kostengünstige Herstellung des lösungsgemäßen Katheters, indem unter Verwendung gängiger Schneideverfahren wenigstens ein helikal umlaufender Trennspalt in Form eines bloßen Einschnittes längs des Katheterabschnittes eingebracht wird. Durch den Schneidevorgang erfährt der Katheter keinerlei bzw. so gut wie keinen Materialabtrag, so dass sich zwei längs des Katheterabschnittes unmittelbar einander anliegende Trennspaltschnittflächen ausbilden, die jeweils von einer radial zum Katheter innen und außen liegenden scharfen Schnittkante begrenzt werden. Vor allem die radial aussen liegende Schnittkante unterstützt eine vollständige Durchtrennung von zu ablatierendem Thrombusmaterial von der Gefäßinnenwand.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1a, b: schematisierte Darstellung eines lösungsgemäß ausgebildeten Katheters a) in einem Zustand frei von äußeren Kräften sowie b) unter Einwirkung eines längs des Katheters wirkenden Drehmomentes,
- Fig. 2a bis e: Varianten für einen Fixiermechanismus,
- Fig. 3: innerhalb eines Hohlgefäßes gegenüber eines Thrombus platzierter Katheterabschnitt, und
- Fig. 4a, b: schematisierte Darstellung einer weiteren Ausführungsform eines lösungsgemäß ausgebildeten Katheters a) in einem Zustand frei von äußeren Kräften sowie b) unter Einwirkung eines längs des Katheters wirkenden Drehmomentes,

### Wege zur Ausführung der Erfindung, gewerbliche Verwendbarkeit

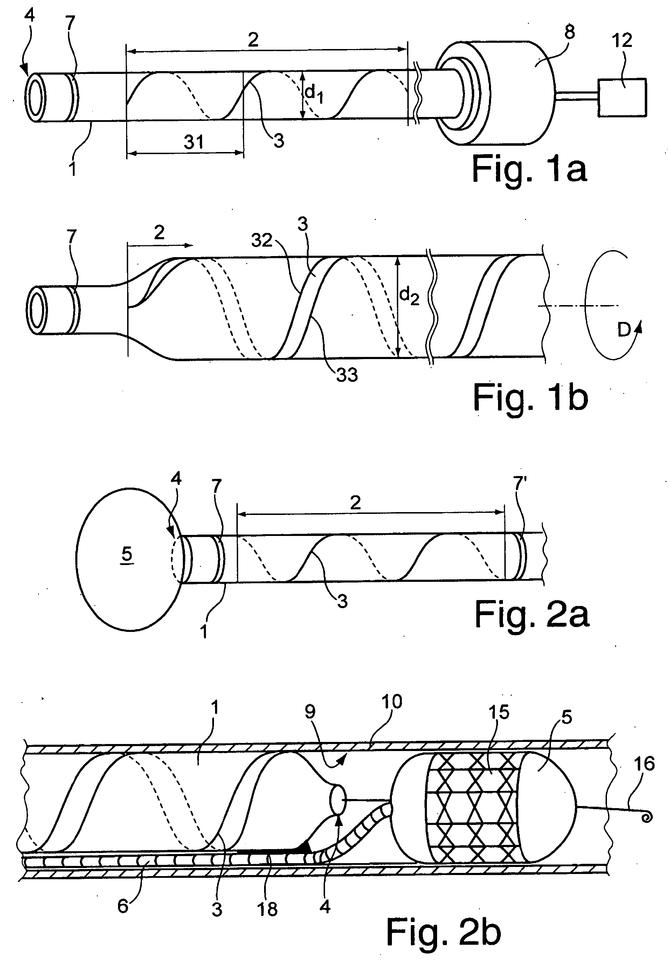

Figur 1 a zeigt stark schematisiert einen lösungsgemäß ausgebildeten Katheter 1, der rohr- bzw. schlauchförmig ausgebildet ist und dessen Katheterwand aus einem biokompatiblen, elastischen Elastomer besteht. Längs des Katheters 1 ist wenigstens ein Katheterabschnitt 2 vorgesehen, in dem ein helikal umlaufender Trennspalt 3 eingebracht ist, der mit Hilfe eines nichtmaterialabtragenden Trennverfahrens, vorzugsweise im Wege eines Schneideverfahrens in Form eines die Katheterwand vollständig durchtrennenden Einschnittes hergestellt wird. Durch den helikal umlaufenden Trennspalt 3 erfährt der Katheterabschnitt 2 keine nach außen optisch in Erscheinung tretende Oberflächenirritation, vielmehr nimmt der als helikale Einschnitt ausgebildete Trennspalt 3 eine geschlossene, d.h. spaltöffnungsfreie Form an. Im in Figur 1 a dargestellten Fall weist der helikale Trennspalt 3 zweieinhalb Windungen 31 mit jeweils konstanter Steigung auf.

Wird der in Figur 1 a dargestellte mit wenigstens einem helikal umlaufenden Trennspalt 3 versehene Katheter an seiner distalen Katheterspitze 4 fixiert, und wird darüber hinaus der Katheter 1 proximalseitig mit einem Drehmoment D beaufschlagt, dessen Drehrichtung entgegengesetzt zur Windungsrichtung des in den Katheter 1 eingebrachten helikal umlaufenden Trennspaltes 3 orientiert ist, so öffnet sich der Trennspalt 3, wobei die sich gegenüberliegenden Schnittflächen 32, 33 des Trennspaltes 3 voneinander beabstanden, wie dies in Figur 1 b illustriert ist. Zugleich weitet sich der Katheteraußendurchmesser d₂ signifikant aus, mit d₂ > d₁, wie dies in der Bilddarstellung gemäß Figur 1 b im Vergleich zu Figur 1 a ersichtlich ist. Mit der torsionsbedingten Spaltöffnung des Trennspaltes 3 geht auch eine Längenausdehnung des Katheters 1 im Bereich des Katheterabschnittes 2 einher.

Wird hingegen das Drehmoment D auf Null reduziert, so formt sich der in Figur 1 b dargestellte aufgeweitete Katheterabschnitt 2 aufgrund materialinhärenter elastischer Rückstellkräfte selbständig in die in Figur 1 a dargestellte Ausgangsform zurück. Hierdurch schließt sich der geöffnete Trennspalt 3, wie dies in Figur 1 a dargestellt ist.

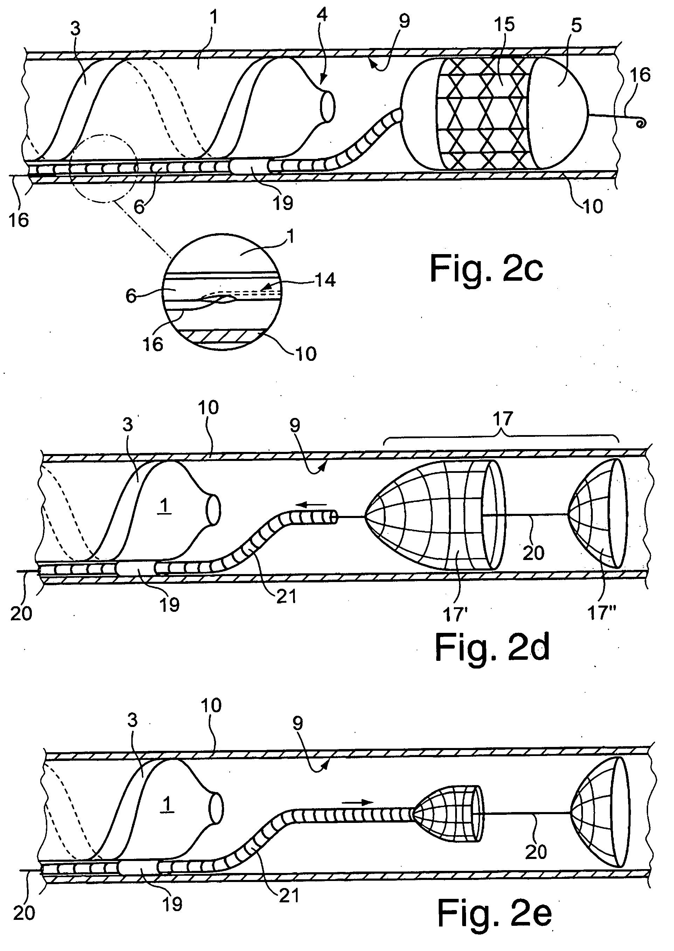

Der vorstehend beschriebenen Mechanismus des Öffnens und Wiederverschließens des sich helikal in Längsrichtung um den Katheterabschnitt 2 umlaufenden Trennspaltes 3 eignet sich zum Abtrennen wandständiger Thromben innerhalb von blutdurchströmten Körpergefäßen. Für den Ablationsvorgang gilt es dafür Sorge zu tragen, dass der intravaskulär gegenüber eines Thrombus positionierte Katheter 1 an seinem distalen Ende 4 oder zumindest distalseitig zum Katheterabschnitt 2 mit Hilfe eines geeigneten Fixiermittels relativ zum Körpergefäß fixiert wird. Insbesondere gilt es bei der Fixierung darauf zu achten, dass der Katheter 1 drehfest um seine Längsachse innerhalb des Hohlgefäßes fixiert wird, um das zum Öffnen des Trennspaltes erforderliche Drehmoment aufzunehmen bzw. gegenüber dem Hohlgefäß abzustützen. Als geeignetes Fixiermittel dient hierzu vorzugsweise ein am distalen Ende 4 des Katheters 1 angebrachter, dilatierbarer Ballon 5, siehe Figur 2 a, der über ein entsprechend innerhalb des Lumens des Katheters 1 verlaufenden Versorgungskanal (nicht dargestellt) mit einem geeigneten Inflatiermedium, wie Luft oder ein flüssiges Medium, gefüllt werden kann. Der Ballon 5 kann entweder fest am distalen Ende des Katheters 1 angebracht oder separat zum Katheter 1 durch das innere Lumen des Katheters 1 distalseitig vorgeschoben werden. Distalseitig und proximalseitig angrenzend zum Katheterabschnitt 2, längs dem der helikal umlaufende Trennspalt 3 angebracht ist, sind röntgendichte Markierungen 7, 7' in am Katheter 1 ein- oder angebracht.

Figur 2b zeigt einen Teil-Längsschnitt eines innerhalb eines Hohlgefäßes 10 platzierten Katheters 1, der längs eines Katheterabschnittes 2 über einen helikal umlaufenden Trennspalt 3 verfügt. Der Trennspalt 3 endet kurz vor dem distalen Ende 4 des Katheters 1. Zumindest in diesem Bereich nahe des distalen Endes 4 ist der Katheter 1 mit einem parallel außerhalb des Katheters 1 geführten Ballonkatheter 6 verbunden. Die Verbindung 18 zwischen Katheter 1 und Ballonkatheter 6 ist derart ausgebildet, dass der Ballonkatheter 6 zumindest in Umfangsrichtung des Katheters 1 fest verbunden ist, bspw. mittels einer Kleb- oder Stoffschlussverbindung.

Der Ballonkatheter 6 umschließt ein inneres Lumen, das proximalseitig mit einer Inflatiereinrichtung (nicht dargestellt) verbindbar, mit der der distalseitig am Ballonkatheter 6 angebrachte Ballon 5 inflatiert bar ist. Der Ballon 5 ist vorzugsweise mit einen netzförmigen, radial aufweitbaren Stent 15 kombiniert, den der Ballon 5 im inflatierten Zustand inwandig gegen die Körpergefäßinnenwand 9 kraftbeaufschlagt zu pressen vermag, um auf diese Weise zum einen für einen drehfesten Halt des Katheters 1 innerhalb des Körpergefäßes 10 zu sorgen, zum anderen den Stent 15 nach entsprechender Deflatierung des Ballons 5 und proximalseitiger Entnahme zu Zwecken einer lokalen Gefäßerweiterung vor Ort zu belassen.

Während der inflatierte Stentballon 5/15 gegen die Gefäßinnenwand 9 gepresst wird und somit das Gefäß lokal verschließt, typischerweise dauert die Balloninflation 1 bis 5 Minuten, wird der helikal eingeschnittene Katheterabschnitt des Katheters 1 durch Applikation eines Drehmomentes längs des , 16Katheters 1 von Seiten des proximalen Katheterendes torsional verformt, wobei sich der Trennspalt 3 öffnet. Vermittels eines proximalseitig längs des Katheterlumens angelegten Aspirationsunterdruckes wird Katheterseitig zum Ballon 5 Gefäßwandständiger Thrombus aspiriert und zwar über den rotierten und damit geöffneten Trennspalt 3 des helikal eingeschnittenen Katheterabschnitts. Ebenso verläuft zu Zwecken der intrakorporalen Navigation des Katheters 1 ein in einem innerhalb des Katheterlumens verlaufenden Zusatzlumen geführter Führungsdraht 16, in Art einer "over the wire" Konfiguration, der distalseitig aus dem Katheter 1 herausragt und seitlich am dillatierten Ballon 5 vorbei oder durch diesen durch eine entsprechende Ausnehmung hindurch verläuft.

Anzumerken ist, dass der intravasale Katheterabschnitt, längs dem der helikal umlaufende Trennspalt 3 eingebracht ist, typischerweise eine Länge von 2-50 cm aufweist und vollständig intrakvaskulär positioniert ist, so dass durch den helikal umlaufenden Trennspalt 3 kein anderes Gewebematerial oder gar Luft aspiriert werden kann.

In einer weiteren Ausführungsform, die in Figur 2c gezeigt ist, ist außerhalb längs des Katheters 1 ein Ballonkatheter 6 längsbeweglich geführt. Zur drehfesten Verbindung des Ballonkatheters 6 relativ zum Katheter1 sieht dieser an seinem distalen Endbereich ein äußeres, kurzes Zusatzlumen 19 vor, durch das der Ballonkatheter 6 geführt ist.

Mit Hilfe eines Führungsdrahtes 16 wird der gesamte Katheter 1 samt des daran längsbeweglich angebrachten Ballonkatheters 6 intravasal geführt und relativ zu einem wandständigen Thrombus positioniert, wobei der Ballon 5 zusätzlich distalwärts zum Katheter 1 vorgeschoben werden kann, um den Ballon 5 in einem Abstand zum distalen Ende 4 des Katheters 1 zu platzieren und in dieser Position zu inflatieren. Gleichsam wie im Ausführungsbeispiel gemäß Figur 2b ist der Ballon 5 als Stentballon 5/15 ausgeführt.

Der Führungsdraht 16 verläuft in diesem Fall in Form einer Monorail-Konfiguration, d.h. weitgehend außerhalb des Katheters1. Lediglich am distalen Bereich des Katheters 1 tritt der Führungsdraht 16 in ein längs zum Katheter 1 geführtes Lumen ein, das im illustrierten Fall als Zusatzlumen 14 innerhalb des Ballonkatheters 6 verläuft. Siehe hierzu die Detaildarstellung zur Figur 2c.

In Figur 2 d ist eine weitere Ausführungsform gezeigt, die anstelle des vorstehenden Ballonkatheters bzw. Stentballons als Fixiermittel einen Katheter 20 mit selbstexpandierender Filtersegelanordnung 17 vorsieht, der gleichsam am distalen Ende 4 des Katheters 1 durch ein an diesem fest angebrachten Zusatzlumen 19 längsbeweglich hindurchgeführt ist. Die Filtersegelanordnung 17 weist zwei längs eines Drahtes 20 angeordnete, selbstexpandierende Filtersegel 17', 17" auf, die vorzugsweise körbchenartig ausgebildet sind und aus einem Formgedächtnismaterial, bspw. einer Ni-Ti-Legierung bestehen. Zum intrakorporalen Einführen der gesamten Katheteranordnung, d.h. des Katheters 1 mit dem daran angebrachten Katheter 21, befinden sich beide Filtersegel 17', 17" innerhalb des Katheters 21 in einem zusammengefalteten Zustand. Nach entsprechender intravaskulärer Positionierung des Katheters 1 über einen Führungsdraht, vgl. Fig. 2c, kann die Filtersegelanordnung 17 durch Zurückziehen der hohlförmigen Katheters 20 relativ zum Draht 20 mit der daran angebrachten Filtersegelanordnung 17 aufgrund der dem Filtersegelmaterial innewohnenden Formgedächtniseffektes sequentiell entfaltet werden, d.h. zunächst entfaltet sich das distalseitige, kleiner ausgebildete Filtersegel 17" und schmiegt sich mit seinem schirmartigen Umfangsrand an die Innenwand 9 des Körpergefäßes an. Durch weiteres Zurückziehen entfaltet sich ebenso das etwas größer ausgebildete Filtersegel 17' im Abstand zum kleineren Filtersegel 17". Im entfalteten Zustand beider Segel, die sich jeweils an die Gefäßinnenwand 9 anschmiegen, wird sichergestellt, dass keinerlei ablatiertes Thrombusmaterial unkontrolliert im Körpergefäß umher vagabundieren kann, da sämtliches von der Gefäßinnenwand 9 abgetrenntes Gewebematerial entweder durch den geöffneten Trennspalt 3 des Katheter 1 aspiriert oder mit der Filtersegelanordnung 17 aufgefangen wird. Zum anderen gewährleisten beide aufgespannten Filtersegel 17", 17' den für das Einwirken des Drehmomentes längs des Katheters 1 zum Öffnen des helikal umlaufenden Trennspalt 3 erforderliche, gegenüber der Gefäßinnenwand 9 abstützende Halte- bzw. Gegenmoment

Nach Beendigung des Gewebeabtrages wird der Katheter 21 relativ zum Draht 20 distalseitig nach vorn geschoben, wodurch sich der Katheter 21 zunächst über das größere Filtersegel schiebt und dieses Regenschirmartig zusammenfaltet, siehe Figur 2e. Dabei werden sämtliche von dem Filtersegel 17' eingefangenen Gewebepartikel sicher im Inneren des Katheters 21 verwahrt. Durch weiteres Vorschieben des Katheters 21 wird das kleinere Filtersegel 17" in der gleichen Weise innerhalb des Katheters 21 verstaut.

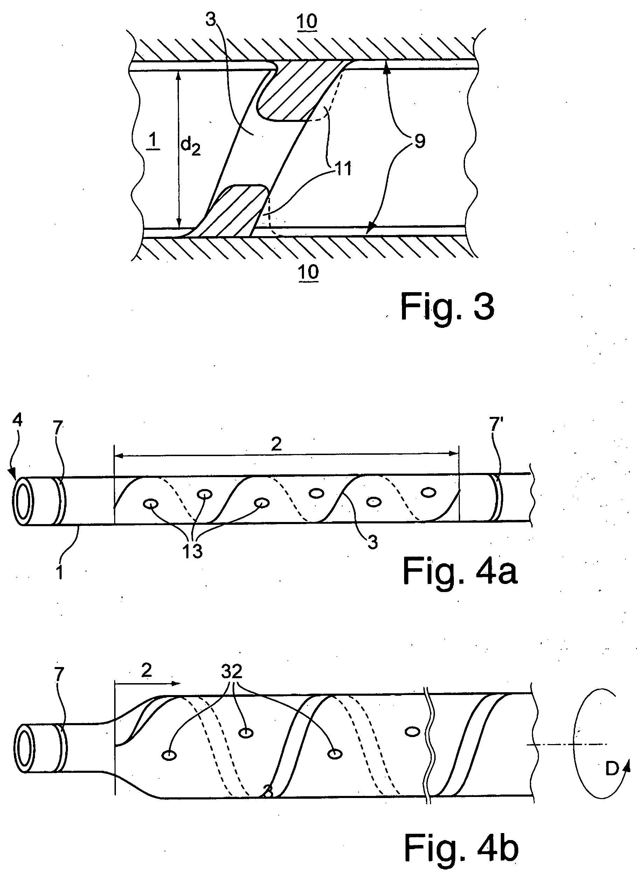

Ferner verfügt der Katheters 1 in allen denkbaren Ausführungsformen zum Zwecke einer erleichterten intrakorporalen Navigation des Katheters über wenigstens eine röntgendichte Markierung 7, anhand der ein Operateur die genaue Lage des Katheters 1 mit Hilfe geeigneter röntgentechnischer Beobachtungsverfahren überwachen zu kann. Vorzugsweise begrenzen zwei röntgendichte Markierungen 7, 7' (siehe Fig. 4a) den Katheterbereich 2, längs dem der helikal umlaufende Trennspalt 3 eingebracht ist, distalseitig und proximalseitig, so dass ein Operateur stets Kenntnis von der intrakorporalen Lage des gesamtem Katheterabschnittes 2 während des Eingriffes hat.

Nach entsprechender Positionierung und Verankerung des Katheters 1 innerhalb des Hohlgefäßes gilt es den Katheter 1 längs seines wenigstens einen helikal umlaufenden Trennspaltes 3 zu öffnen. Hierzu übt ein Operateur proximalseitig am Katheter entweder manuell oder mit Hilfe einer geeigneten Drehvorrichtung 8, siehe Fig. 1a, ein Drehmoment D mit einem entgegen zur Windungsrichtung des helikal umlaufenden Trennspaltes 3 orientierten Drehsinn auf den Katheter 1 aus. Durch die Trennspaltöffnung vergrößert sich zugleich auch der Katheteraußendurchmesser d₂ und schmiegt sich presskraftbeaufschlagt an die Innenwand 9 eines Hohlgefäßes 10 an, siehe hierzu Fig. 3. In den geöffneten Trennspalt 3 vermag nun wandständiges Thrombusmaterial 11 einzudringen. Dieser Vorgang des Eindringens von Thrombusmaterial durch den aufgespreizten Trennspalt 3 in das Lumen des Katheters 1 kann in vorteilhafter Weise durch Applikation eines Aspirationsunterdruckes längs des Katheterlumens unterstütz werden. Hierzu weist der Katheter 1 im proximalen Bereich eine fluiddichte Verbindungsstruktur zum Anbringen einer geeigneten Unterdruckquelle 12 auf (siehe Fig. 1a).

Nach entsprechendem Beseitigen des Drehmomentes D schließt sich der in Figur 3 in geöffneter Darstellung gezeigte Trennspalt 3 und schert zugleich an der Innenwand 9 verbleibendes in das Innere des Katheters 1 hineinreichendes Thrombusmaterial 11 von der Innenwand 9 des Hohlgefäßes 10 ab. Durch proximalseitige Entnahme kann auf diese Weise das Thrombusmaterial sicher im Inneren des Katheters extrakorporal verbracht werden.

In den Figuren 4a, b ist ein weiteres alternatives Ausführungsbeispiel zur Ausgestaltung des lösungsgemäßen Katheters 1 gezeigt, das zusätzlich zu dem helikal umlaufenden Trennspalt 3 längs des Katheterabschnittes 2 die Katheterwand 3 vollständig durchsetzende Wandöffnungen 13 vorsieht, die im Unterschied zum Trennspalt 3 im Wege eines Material abtragenden Verfahrens in die Katheterwand eingebracht sind, bspw. im Wege eines Stanzverfahrens, eines mechanischen oder thermischen Material abtragenden Abrassivverfahrens. Die Wandöffnungen 13 behalten ihre Öffnungsgeometrie unabhängig von der elastischen Formänderung des Katheters 1 unverändert bei und ermöglichen in Verbindung mit einer Unterdruckapplikation im Inneren des Katheters 1 zusätzliche Möglichkeiten zur sicheren Ablation und Verwahrung von abgetrennten, wandständigen Thrombusmaterial im Inneren des Katheters 1.

Die Dimensionierung des lösungsgemäßen Katheters 1 ist abhängig von den geometrischen Gegebenheiten intrakorporaler Hohlgefäße. So bemisst sich die axiale Länge des Katheterabschnittes 2, längs dem der wenigstens eine helikal umlaufende Trennspalt 3 vorgesehen ist, in einer Größenordnung zwischen 1 cm und 1 m. Selbstverständlich ist es möglich, längs des Katheters 1 in unterschiedlichen axialen Bereichen, die axial voneinander beabstandet sind, jeweils einen oder mehrere helikal umlaufende Trennspalte einzubringen.

Geeignete Katheteraußendurchmesser, die der Katheter in einem kräftefreien Zustand einnimmt, liegen typischerweise zwischen 1 mm und 25 mm, wobei der Katheter eine Katheterwandstärke zwischen 0,1 mm und 2,5 mm aufweist. Im Falle der drehmomentbedingten Aufweitung des Katheters weist der geöffnete Trennspalt typischerweise Spaltweiten zwischen 0,1 mm und 50 mm, vorzugsweise zwischen 0,5 mm und 10 mm auf. Die in den Figuren 4 a und b illustrierten zusätzlichen Wandöffnungen 13 verfügen typischerweise über Öffnungsweiten von 0,1 mm bis 20 mm.

Der lösungsgemäße Katheter 1 bedarf somit zum Zwecke der Ablation innwandiger Thrombusmaterialablagerungen keiner zusätzlich zum Katheter handzuhabenden Schneidewerkzeuge oder den Katheter radial aufweitenden Verdrängungskörper, vielmehr ist der lösungsgemäß ausgebildete Katheter intravasal zu fixieren und ausschließlich einem Drehmoment zu unterwerfen, durch das der Katheter seine volle Funktionalität entfaltet.

### Bezugszeichenliste

- 1: Katheter
- 2: Katheterabschnitt
- 3: helikal umlaufender Trennspalt
- 31: Windung
- 32,33: Schnittflächen
- 4: distales Katheterende
- 5: Ballon
- 6: Ballonkatheter
- 7, 7': Röntgendichte Markierung
- 8: Drehmotor
- 9: Gefäßinnenwand
- 10: Hohlgefäß
- 11: Thrombusmaterial
- 12: Unterdruckquelle
- 13: Wandöffnung
- 14: Zusatzlumen
- 15: Stent
- 16: Führungsdraht
- 17: Filtersegelanordnung
- 17', 17": Filtersegel
- 18: Verbindung
- 19: Zusatzlumen
- 20: Draht
- 21: Katheter

## Patentansprüche

1. Vorrichtung zum Ablösen wandständiger Thromben aus einem Körpergefäß mit einem Katheter (1), längs dessen Katheterlängserstreckung wenigstens ein Katheterabschnitt (2) vorgesehen ist, in dessen zuordenbaren Katheterwand wenigstens eine die Katheterwand vollständig durchsetzende Wandöffnung vorgesehen ist,
wobei die Katheterwand längs des wenigstens einen Katheterabschnitts (2) aus einem elastisch verformbaren Material besteht, **dadurch gekennzeichnet, dass** die wenigstens eine Wandöffnung in Form eines die Katheterwand wenigstens abschnittsweise längs des Katheterabschnitts helikal umlaufenden Trennspaltes (3) ausgeführt ist,
dass der Katheter (1) distalseitig zum helikal umlaufenden Trennspalt (3) ein Fixiermittel vorsieht, das eine wiederlösbare Fixierung des Katheters (1) an einem Körpergefäß (10) ermöglicht,
dass der Katheter (1) ausschließlich durch einen äußeren mechanischen Zwang, in Form eines auf den Katheter torsional einwirkenden Drehmomentes, von einem Zustand mit relativ kleinerem Katheteraußendurchmesser in einen Zustand mit relativ größerem Katheteraußendurchmesser im Wege einer elastischen Formänderung überführbar ist, wobei sich der helikal umlaufende Trennspalt (3) von einem geschlossenen Spaltzustand in einen geöffneten Spaltzustand aufweitet, und
dass unter Wegfall des äußeren Zwangs der Katheter (1) durch materialinhärente, elastische Rückstellkräfte den Zustand mit dem relativ kleineren Katheteraußendurchmesser selbständig einnimmt und der Trennspalt (3) den geschlossenen Spaltzustand einnimmt.

2. Vorrichtung nach Anspruch 1,
**dadurch gekennzeichnet, dass** die Katheterwand des wenigstens einen Katheterabschnittes (2) schlauchförmig ausgebildet ist, ein inneres Katheterlumen umfasst, und
dass das elastisch verformbare Material ein Elastomer ist.

3. Vorrichtung nach Anspruch 1 oder 2,
**dadurch gekennzeichnet, dass** der wenigstens eine Katheterabschnitt (2) in Katheterlängserstreckung 1 cm bis 100 cm lang ausgebildet ist, einen elastischen Abschnitt mit einem Katheteraußendurchmesser von 1 mm bis 25 mm sowie eine Katheterwandstärke ohne Einwirkung des äußeren Zwangs zwischen 0,1 mm und 2,5 mm aufweist.

4. Vorrichtung nach einem der Ansprüche 1 bis 3
**dadurch gekennzeichnet, dass** der helikal umlaufende Trennspalt (3) längs des wenigstens einen Katheterabschnittes (2) wenigstens einen Wendelgang aufweist.

5. Vorrichtung nach einem der Ansprüche 1 bis 4,
**dadurch gekennzeichnet, dass** der wenigstens eine helikal umlaufende Trennspalt (3) längs des wenigstens einen Katheterabschnittes (2) als ein die Katheterwand vollständig durchtrennender Einschnitt ausgebildet ist, mit zwei sich im geschlossenen Spaltzustand unmittelbar berührenden Schnittflächen (32, 33).

6. Vorrichtung nach einem der Ansprüche 1 bis 5,
**dadurch gekennzeichnet, dass** der wenigstens eine helikal umlaufende Trennspalt (3) längs des wenigstens einen Katheterabschnittes (2) eine konstante Steigung aufweist.

7. Vorrichtung nach einem der Ansprüche 1 bis 6,
**dadurch gekennzeichnet, dass** längs der Katheterlängserstreckung wenigstens ein erster Katheterabschnitt und axial zu diesem beabstandet ein zweiter Katheterabschnitt vorgesehen sind, und
dass in den wenigstens zwei Katheterabschnitten (2) jeweils wenigstens ein helikal umlaufender Trennspalt (3) mit jeweils gleicher Wendelrichtung eingebracht ist.

8. Vorrichtung nach einem der Ansprüche 1 bis 7,
**dadurch gekennzeichnet, dass** das Fixiermittel in Form eines dillatierbaren Ballons (5) ausgebildet ist, der distalseitig zu dem wenigstens einen helikal umlaufenden Trennspalt (3) am Katheter (1) angebracht ist oder durch den Katheter (1) hindurch distalseitig zu diesem positionierbar ist, und
dass der Ballon (5) über ein innerhalb oder außerhalb des Katheterlumens verlaufendes Zusatzlumen inflatierbar ist.

9. Vorrichtung nach einem der Ansprüche 1 bis 8,
**dadurch gekennzeichnet, dass** das Fixiermittel in Form eines mechanisch aufspreizbaren Körpers ausgebildet ist, der distalseitig zu dem wenigstens einen helikal umlaufenden Trennspalt (3) am Katheter (1) angebracht ist oder durch den Katheter (1) hindurch distalseitig zu diesem positionierbar ist, und
dass der aufspreizbare Körper über einen, durch ein innerhalb oder außerhalb des Katheterlumens, zumindest abschnittsweise verlaufendes Zusatzlumen geführter Betätigungsdraht (20) spreizbar und zusammenfaltbar ist.

10. Vorrichtung nach Anspruch 9,
**dadurch gekennzeichnet, dass** der aufspreizbare Körper netzartig ausgebildet ist und in einem aufgespreizten Zustand für ein durch das Körpergefäß strömendes Fluid eine Filterfunktion besitzt.

11. Vorrichtung nach einem der Ansprüche 1 bis 10,
**dadurch gekennzeichnet, dass** der Katheter proximalseitig einen Anschluss vorsieht, an den eine Unterdruckquelle (12) in Form einer Aspirationseinheit und/öder ein motorischer Drehantrieb (8) anschließbar ist/sind.

12. Vorrichtung nach einem der Ansprüche 1 bis 11,
**dadurch gekennzeichnet, dass** im Bereich der dem Katheter (1) zuordenbaren Katheterspitze und/oder längs des wenigstens einen Katheterabschnittes (2) wenigstens eine röntgendichte Markierung (7, 7') angebracht ist.

13. Vorrichtung nach einem der Ansprüche 1 bis 12,
**dadurch gekennzeichnet, dass** von der dem Katheter (1) zuordenbaren Katheterspitze bis zum wenigstens einen Katheterabschnitt (2) an der Katheteraußenseite über eine Länge von 20-50 mm ein weiteres Lumen zur Führung des Katheters über einen Führungsdraht (16) angebracht ist.

14. Vorrichtung nach einem der Ansprüche 5 bis 13,
**dadurch gekennzeichnet, dass** sich die Schnittflächen des wenigstens einen helikal umlaufenden Einschnittes beim Überführen des Katheters (1) vom Zustand mit dem relativ kleineren Katheteraußendurchmesser in den Zustand mit dem relativ größeren Katheteraußendurchmesser durch den äußeren Zwang voneinander beabstanden und dabei einen, helikal umlaufenden Spalt einschließen mit einer Steigung, die größer ist als eine dem helikal umlaufenden Einschnitt zuordenbaren Steigung im Zustand mit dem relativ kleineren Katheteraußendurchmesser.

15. Vorrichtung nach Anspruch 14,
**dadurch gekennzeichnet, dass** der helikal umlaufende Spalt eine größte Spaltbreite b aufweist, für die gilt 0,1 mm ≤ b ≤ 50 mm, 0,5 mm ≤ b ≤ 10 mm aufweist.

## Claims

1. A device for detaching parietal thrombi from a vessel of the body, having a catheter (1) with at least one catheter section (2) provided along the longitudinal direction of the catheter, the catheter wall which is associated therewith being provided with at least one wall opening which penetrates completely through the catheter wall,
wherein the catheter wall consists of a resiliently deformable material along the at least one catheter section (2),
**characterized in that** the at least one wall opening is in the form of a circumferential helical separating slit (3) in at least a part of the catheter wall along the catheter section,
**in that**, distally to the circumferential helical separating slit (3), the catheter (1) is provided with a fixing means which permits releasable fixing of the catheter (1) to a vessel of the body (10),
**in that** the catheter (1) can be converted exclusively by an external mechanical force in the form of a turning moment acting torsionally on the catheter, from a state with a relatively smaller external catheter diameter into a state with a relatively larger external catheter diameter by means of a resilient change in shape, whereby the circumferential helical separating slit (3) expands from a closed state of the slit into an open state of the slit, and
**in that** when the external force ceases to act, the catheter (1) autonomously assumes the state with the relatively smaller external catheter diameter by means of the inherent resilient restoring forces of the material and the separating slit (3) assumes the closed state of the slit.

2. The device as claimed in claim 1,
**characterized in that** the catheter wall of the at least one catheter section (2) is in the form of a tube and comprises an interior catheter lumen, and **in that** the resiliently deformable material is an elastomer.

3. The device as claimed in claim 1 or claim 2,
**characterized in that** the at least one catheter section (2) is 1 cm to 100 cm in length in the longitudinal direction of the catheter and has a resilient section with an external catheter diameter of 1 mm to 25 mm as well as a catheter wall thickness, in the absence of the action of the external force, of between 0.1 mm and 2.5 mm.

4. The device as claimed in one of claims 1 to 3,
**characterized in that** the circumferential helical separating slit (3) along the at least one catheter section (2) has at least one helical turn.

5. The device as claimed in one of claims 1 to 4,
**characterized in that** the at least one circumferential helical separating slit (3) along the at least one catheter section (2) is formed as an incision which penetrates completely through the catheter wall with two cut surfaces (32, 33) which are in direct contact when the slit is in the closed state.

6. The device as claimed in one of claims 1 to 5,
**characterized in that** the at least one circumferential helical separating slit (3) along the at least one catheter section (2) has a constant pitch.

7. The device as claimed in one of claims 1 to 6,
**characterized in that** at least a first catheter section and a second catheter section which is axially offset to the first section are provided in the longitudinal direction of the catheter, and
**in that** at least one circumferential helical separating slit (3) each with the same direction of turn is provided in each of the at least two catheter sections (2).

8. The device as claimed in one of claims 1 to 7,
**characterized in that** the fixing means is in the form of a dilatable balloon (5) which is mounted on the catheter (1) distally to the at least one circumferential helical separating slit (3) or can be positioned distally thereto through the catheter (1),
and
**in that** the balloon (5) can be inflated via an additional lumen which runs inside or outside the catheter lumen.

9. The device as claimed in one of claims 1 to 8,
**characterized in that** the fixing means is in the form of a body which can be mechanically splayed out which is mounted on the catheter (1) distally to the at least one circumferential helical separating slit (3) or can be positioned distally thereto through the catheter (1), and
**in that** the splayable body can be deployed and collapsed via an actuating wire (20) which is guided at least in part in an additional lumen which runs inside or outside the catheter lumen.

10. The device as claimed in claim 9,
**characterized in that** the splayable body is in the form of a net and in the splayed out state it functions as a filter for fluid flowing through the vessel of the body.

11. The device as claimed in one of claims 1 to 10,
**characterized in that** the catheter is provided with a proximal connector to which a vacuum source (12) in the form of a suction unit and/or a motorized rotary actuator (8) can be connected.

12. The device as claimed in one of claims 1 to 11,
**characterized in that** at least one radiopaque marking (7, 7") is provided in the region of the catheter tip associated with the catheter (1) and/or along the at least one catheter section (2).

13. The device as claimed in one of claims 1 to 12,
**characterized in that** a further lumen for guiding the catheter via a guide wire (16) is attached from the catheter tip associated with the catheter (1) to the at least one catheter section (2) on the exterior of the catheter over a length of 20 - 50 mm.

14. The device as claimed in one of claims 5 to 13,
**characterized in that** when converting the catheter (1) from the state with the relatively smaller external catheter diameter into the state with the relatively larger external catheter diameter, the cut surfaces of the at least one circumferential helical incision are separated from each other by the external force and thus enclose a circumferential helical slit having a pitch which is larger than the pitch associated with the circumferential helical incision in the state with the relatively smaller external catheter diameter.

15. The device as claimed in claim 14,
**characterized in that** the circumferential helical slit has a largest slit width b for which 0.1 mm ≤ b ≤ 50 mm, 0.5 mm ≤ b ≤ 10 mm hold.

## Revendications

1. Dispositif pour détacher des thrombus pariétaux d'un vaisseau, comportant un cathéter (1) le long de l'extension de cathéter de laquelle il est prévu au moins une section de cathéter (2) dans la paroi de cathéter associable de laquelle au moins un orifice de paroi traversant complètement la paroi du cathéter est prévu,
la paroi de cathéter étant composée le long de l'au moins une section de cathéter (2) d'un matériau élastiquement déformable,
**caractérisé en ce que** l'au moins un orifice de paroi est réalisé sous forme d'un intervalle de séparation périphérique de forme hélicoïdale du moins par sections le long de la section de cathéter (3),
qu'il est prévu sur le cathéter (1), au niveau distal par rapport à l'intervalle de séparation périphérique de forme hélicoïdale (3), un moyen de fixation qui permet une fixation dissociable du cathéter (1) à un vaisseau (10),
que le cathéter (1) peut être passé exclusivement par une contrainte mécanique extérieure, sous forme d'un couple de rotation agissant en torsion sur le cathéter (3), d'un état où le diamètre extérieur du cathéter est relativement réduit à un état où le diamètre du cathéter est relativement important au moyen d'une modification élastique de la forme, l'intervalle de séparation périphérique de forme hélicoïdale (3) s'élargissant d'un état d'intervalle fermé jusqu'à un état d'intervalle ouvert, et
que, en l'absence de la contrainte extérieure, le cathéter (1), par des forces de rappel élastiques inhérentes au matériau, prend automatiquement l'état où le diamètre extérieur du cathéter est relativement réduit et l'intervalle de séparation (3) prend l'état de fermeture de l'intervalle.

2. Dispositif selon la revendication 1,
**caractérisé en ce que** la paroi de cathéter de l'au moins une section de cathéter (2) a une conformation tubulaire, comprend un lumen interne de cathéter et que le matériau élastiquement déformable est un élastomère.

3. Dispositif selon la revendication 1 ou 2,
**caractérisé en ce que** l'au moins une section de cathéter (2) est réalisée avec une extension longitudinale du cathéter de 1 cm à 100 cm de long, présente une section élastique ayant un diamètre extérieur de cathéter de 1 mm à 25 mm et une épaisseur de paroi de cathéter de 0,1 mm et 2,5 mm sans action de la contrainte extérieure.

4. Dispositif selon une des revendications 1 à 3,
**caractérisé en ce que** l'intervalle de séparation périphérique de forme hélicoïdale (3) présente au moins un pas de spires le long de l'au moins une section de cathéter (2).

5. Dispositif selon une des revendications 1 à 4,
**caractérisé en ce que** l'au moins un intervalle de séparation de forme hélicoïdale (3) est réalisé le long de l'au moins une section de cathéter (2) sous forme d'une incision coupant entièrement la paroi du cathéter (32, 33) et comportant deux surfaces de coupe se touchant directement en état fermé de l'intervalle.

6. Dispositif selon une des revendications 1 à 5,
**caractérisé en ce que** l'au moins un intervalle de séparation de forme hélicoïdale (3) présente une pente constante le long de l'au moins une section de cathéter (2).

7. Dispositif selon une des revendications 1 à 6,
**caractérisé en ce que**, le long de l'extension longitudinale du cathéter, il est prévu au moins une première section de cathéter et, espacée axialement de celle-ci, une seconde section de cathéter et
que, dans les au moins un deux sections de cathéter (2), respectivement au moins un intervalle de séparation périphérique de forme hélicoïdale (3) est prévu respectivement avec le même sens de spires.

8. Dispositif selon une des revendications 1 à 7,
**caractérisé en ce que** le moyen de fixation se présente sous forme d'un ballon dilatable (5) qui est installé au niveau distal par rapport à l'au moins un intervalle de séparation périphérique de forme hélicoïdale (3) au niveau du cathéter (1) ou est positionnable à travers le cathéter (1) au niveau distal par rapport à celui-ci est
que le ballon (5) est gonflable par un lumen supplémentaire s'étendant à l'intérieur ou à l'extérieur du lumen de cathéter.

9. Dispositif selon une des revendications 1 à 8,
**caractérisé en ce que** le moyen de fixation se présente sous forme d'un corps pouvant être écarté mécaniquement et qui est installé au niveau distal par rapport à l'au moins un intervalle de séparation périphérique de forme hélicoïdale (3) au niveau du cathéter (1) ou est positionnable à travers le cathéter (1) au niveau distal par rapport à celui-ci et
que le corps écartable est écartable et repliable par un fil d'actionnement (20) guidé dans un lumen supplémentaire s'étendant du moins par sections à l'intérieur ou à l'extérieur du lumen de cathéter.

10. Dispositif selon la revendication 9,
**caractérisé en ce que** le corps écartable est réalisé sous forme de filet et assure, en état écarté, une fonction de filtre pour le fluide s'écoulant dans le vaisseau.

11. Dispositif selon une des revendications 1 à 10,
**caractérisé en ce qu'**il est prévu sur le cathéter au niveau proximal un raccord auquel une source de dépression (12) sous forme d'une unité d'aspiration et/ou d'une commande rotative motorisée (8) peut ou peuvent être raccordées.

12. Dispositif selon une des revendications 1 à 11,
**caractérisé en ce que**, au niveau de la pointe de cathéter pouvant être associée au cathéter (1) et/ou le long de l'au moins une section de cathéter (2), au moins un marquage étanche aux rayons X (7, 7') est apposé.

13. Dispositif selon une des revendications 1 à 12,
**caractérisé en ce que**, depuis la pointe de cathéter associable au cathéter (1) et jusqu'à l'au moins une section de cathéter (2), sur la face extérieure du cathéter, sur une longueur de 20-50 mm, un autre lumen est pratiqué pour le guidage du cathéter au moyen d'un fil de guidage (16).

14. Dispositif selon une des revendications 5 à 13,
**caractérisé en ce que** les surfaces de coupe de l'au moins une incision périphérique de forme hélicoïdale, lors du passage du cathéter (1) de l'état où le diamètre extérieur du cathéter est relativement réduit à l'état ou le diamètre extérieur du cathéter est relativement important, s'écartent l'une de l'autre sous l'effet de la contrainte extérieure et circonscrivent alors une fente périphérique de forme hélicoïdale ayant une pente qui est supérieure à une pente associable à l'incision périphérique de forme hélicoïdale dans l'état où le diamètre extérieur du cathéter est relativement réduit.

15. Dispositif selon la revendication 14,
**caractérisé en ce que** l'intervalle périphérique de forme hélicoïdale présente une largeur d'intervalle maximale b, sachant que 0,1 mm ≤ b ≤ 50 mm, 0,5 mm ≤ b ≤ 10 mm.
